# EUROPEAN PATENT SPECIFICATION

(11) **EP 2 358 214 B1**
(45) Date of publication and mention of the grant of the patent: **02.10.2013**
(21) Application number: 09748799.5
(22) Date of filing: 11.11.2009
(51) Int. Cl.: A23K 1/16, A23K 1/18

(54) **USE OF CANTHAXANTHIN AND/OR 25-OH D3 FOR IMPROVED HATCHABILITY IN POULTRY**
VERWENDUNG VON CANTHAXANTHIN UND/ODER 25-OH D3 ZUR VERBESSERUNG DES AUSSCHLÜPFENS IN GEFLÜGEL
UTILISATION DE LA CANTHAXANTHIN ET/OU DU 25-OH D3 POUR L'AMELIORATION DE L'ECLOSION CHEZ LA VOLAILLE

(30) Priority: 19.11.2008 EP 08020153
(43) Date of publication of application: 24.08.2011
(73) Proprietor: DSM IP Assets B.V., 6411 TE Heerlen (NL)
(72) Inventor: HERNANDEZ, Jose-Maria, CH-4312 Magden (CH); WEBER, Gilbert, CH-4312 Magden (CH)
(74) Representative: Schwander, Kuno
(86) International application number: PCT/EP2009/064963
(87) International publication number: WO 2010/057811

(56) References cited:
- WO-A2-01/56401
- Robert F, Panheleux- Le Bastard M, Hamelin C, Boulard C: "Effects of canthaxanthin supplementation in the ROSS breeder diet on oxidative stress of chicks", 16th European Symposium on Poultry Nutrition, 26-30/08/2007 , 30 August 2007 (2007-08-30), pages 731-734, XP002565950, Retrieved from the Internet: URL:http://www.cabi.org/animalscience/Uplo ads/File/AnimalScience/additionalFiles/WPS AStrasbourgAug2007/64.pdf [retrieved on 2010-01-28]
- CHANG-HYEUG KIM ET AL: "Effects of dietary xanthophylls and seaweed by-products on growth performance, color and antioxidant properties in broiler chicks", HAN-GUG CHUGSAN SIGPUM HAG-HOEJI - KOREAN SOCIETY FOR FOOD SCIENCE OF ANIMAL RESOURCES, HAN-GUG CHUGSAN SIGPUM HAG-HOEJI, KR, vol. 24, no. 2, 1 June 2004 (2004-06-01), pages 128-134, XP009128663, ISSN: 1225-8563
- PEREZ-VENDRELL AM ET AL: "Influence of source and ratio of xanthophyll pigments on broiler chicken pigmentation and performance", POULTRY SCIENCE, vol. 80, 2001, pages 320-326, XP002565951,
- MCGRAW KJ, KLASING KC: "Carotenoids, immunity, and integumentary coloration in red junglefowl (Gallus Gallus)", THE AUK, vol. 123, no. 4, 2006, pages 1161-1171, XP002565952,
- CUCCO M, GUASCO B, MALACARNE G, OTTONELLI R: "Effects of B-carotene on adult immune condition and antibacterial activity in the eggs of the Grey Partridge, Perdix perdix", COMPARATIVE BIOCHEMISTRY AND PHYSIOLOGY, vol. 147, no. A, 2007, pages 1038-1046, XP002565953,
- ATENCIO A ET AL: "TWENTY-FIVE HYDROXYCHOLECALCIFEROL AS A CHOLECALCIFEROL SUBSTITUTE IN BROILER BREEDER HEN DIETS AND ITS EFFECT ON THE PERFORMANCE AND GENERAL HEALTH OF THE PROGENY", POULTRY SCIENCE, CHAMPAIGN, IL, US, vol. 84, no. 8, 1 August 2005 (2005-08-01) , pages 1277-1285, XP009074311, ISSN: 0032-5791
- SOARES J H ET AL: "THE EFFECTIVENESS OF THE VITAMIN D ANALOG 1 .ALPHA.-OH-D3 IN PROMOTING FERTILITY AND HATCHABILITY IN THE LAYING HEN", POULTRY SCIENCE, CHAMPAIGN, IL, US, vol. 58, no. 4, 1 January 1979 (1979-01-01), pages 1004-1006, XP009074301, ISSN: 0032-5791

## Description

### FIELD OF THE INVENTION

The present invention relates to the use of canthaxanthin and optionally at least one vitamin D metabolite, preferably 25-hydroxy vitamin D3 (25-OH D3), for improving breeder hatchability. The invention also relates to the use of canthaxanthin and optionally 25-hydroxy vitamin D3 in the manufacture of a feed or veterinary composition for lowering embryo mortality in poultry.

### BACKGROUND

To maximize the fertility of the egg and the hatchability of the embryo during the breeding phase, optimal nutritional status of breeders is essential for the effective transfer of nutrients to the embryo.

In accordance with the present invention it has been found that problems in chicken breeding can be eliminated or substantially ameliorated by administering to the animals an effective amount of Canthaxanthin or 25-OH-D3, optionally a combination of both nutrients.

Applicant now surprisingly found that relatively high concentrations of essential nutrients in the embryo such as canthaxanthin or 25-OH-D3 supplied via maternal supplementation are related to improved hatchability, fertility and lower embryo mortality during the first phase of embryo development.

It was found by Atencio et al, Poultry Science 2005; 84: 1277-1285, that 25-OH D3 improves fertility and hatchability, and lowers embryo mortility, in poultry. Robert et al, 16th European Symposium on Poultry Nutrition, 26-30 August 2007, describes the improvement of oxidative status in chicks bom from hens supplemented with canthaxanthin.

Tritsch et al. (US 2003/0170324) disclose a feed premix composition of at least 25-OH D3 in an amount between 5% and 50% (wt/wt) dissolved in oil and an antioxidant, an agent encapsulating droplets of 25-OH D3 and oil, and a nutritional additive (e.g., Vitamin D3). The premix may be added to poultry, swine, canine, or feline food. This composition stabilizes 25-OH D3 against oxidation.

Simoes-Nunes et al. (US 2005/0064018) discloses adding a combination of 25-OH Vitamin D3 and Vitamin D3 to animal feed. In particular, about 10 µg/kg to about 100µg/kg of 25-OH Vitamin D3 and about 200 IU/kg to about 4,000 IU/kg of Vitamin D3 are added to swine feed. This addition improves the pig's bone strength.

Stark et al. (US 5,695,794) disclose adding a combination of 25-OH Vitamin D3 and Vitamin D3 to poultry feed to ameliorate the effects of tibial dyschondroplasia.

Borenstein et al US 5,043,170 discloses the combination of Vitamin D3 and either 1-alpha-hydroxycholecalciferol or 1alpha, 25-dihydroxycholecalciferol to improve egg strength and leg strength in laying hens and older hens.

Fleshner-Barak (WO 03/007916) discloses administration of bisphosphonate compound and natural vitamin D derivative such as 1,25-dihydroxyvitamin D3 or 24,25-dihydroxyvitamin D3, or 25-OH vitamin D3.

Daifotis et al. (WO 03/086415) disclose inhibiting bone resorption by a combination of at least one bisphosphonate compound and from about 100 IU to about 60,000 IU of a no activated metabolite of vitamin D2 and/or vitamin D3.

The aforementioned documents did not teach or suggest that the use of canthaxanthin and 25-OH D3 or a combination thereof would be surprisingly beneficial to improve hatchability.

### DETAILED DESCRIPTION OF THE INVENTION

As used throughout the specification and claims, the following definitions apply:
"Vitamin D metabolite" means any metabolite of Vitamin D as for example 25-hydroxy vitamin D3, 1,25-dihydroxy vitamin D3 or 24,25-dihydroxy vitamin D3.
"25-OH D3" refers specifically to 25-hydroxy vitamin D3.
"Poultry" is meant to include turkeys, ducks and chickens (including but not limited to broiler chicks, layers, breeders).

Canthaxanthin and 25-OH D3 may be obtained from any source, and a composition thereof may be prepared using convenient technology.

In a first aspect, one or more feed compositions suitable for poultry use are provided to administer canthaxanthin optionally in combination with 25-OH D3 as nutrients to improve hatchability, fertility and lower embryo mortality during the first phase of embryo development.

In a second aspect, a poultry feed is provided which comprises from about 10 µg/kg to about 100 µg/kg of 25-OH D3 and/or from about 2 to 100 ppm canthaxanthin, preferably 2 to 10 ppm.

In another aspect, the use of canthaxanthin, optionally in combination with 25-OH D3 for poultry breeders is provided to improve hatchability, fertility and lower embryo mortality during the first phase of embryo development.

The use for improving hatchability in poultry comprises administering to the animal in need of such treatment an amount of about 2 ppm to 100 ppm of canthaxanthin, preferably 2 to 10 ppm, and/or about 10 µg/kg to about 100 µg/kg of 25-OH D3.

In another aspect, a premix composition for poultry feed comprising canthaxanthin and 25-hydroxy vitamin D3 is provided.

Canthaxanthin and 25-hydroxy vitamin D3 are suitably administered together with the food. The term food as used herein comprises both solid and liquid food as well as drinking fluids such as drinking water. Particularly, inventive ingredients can be added as a formulated powder to a premix containing other minerals, vitamins, amino acids and trace elements which is added to regular animal food and thorough mixing to achieve even distribution therein.

In the manufacture of poultry feed in accordance with the invention, from about 2 ppm to 100 ppm, preferably 2 - 10 ppm of canthaxanthin and, if required, from about 10µ/kg to about 100µg/kg of 25-hydroxy vitamin D3 are added to regular poultry food. Alternatively, a food premix may be prepared on the basis of regular food components by adding these active ingredients to such food components in higher concentration.

According to the present invention the canthaxanthin compound is available under the Trademark ROVIMIX® Hy-D® 1.25 % and canthaxanthin under the Trademark CAROPHYLL®Red.

According to the present invention it is further advantageous if the composition also contains one or more of the following ingredients: Vitamin A, Vitamin E, Biotin, copper (e.g. as CuSO₄), zinc (e.g. as ZnSO₄), cobalt (e.g. as CoSO₄), selenium (e.g. as Na₂SeO₃), iodine (e.g. as KI), manganese (e.g. as MnSO₄) and/or calcium (e.g. as CaSO₄).

The following non-limiting Examples are presented to better illustrate the invention.

### Example 1: Effect of Carophyll Red (Canthaxanthin) on the productive and reproductive development of broiler chickens

### Material & Methods

In this study 360 females and 36 male broiler chickens were used, all 45 weeks of age, and of Cobb 500 lineage. The birds were housed together in their respective treatment groups according to body weight and the uniformity of the batch.

### Pre-experiment phase - 37th to 45th week:

In the pre-experiment phase, the birds underwent the recommended handling and feeding practices in the breeders' guide. To assess the fertility of the birds in each box, an incubation session was carried out for one week, and using embryo diagnostics on the eggs that did not hatch, the percentage of fertility in each box was determined. The level of fertility was taken into consideration when distributing the treatments to each of the boxes, so that all treatments had the same level of fertility at the beginning of the study.

### Period of Experimentation - 46th to 66th weeks:

At the start of this period, all the birds were weighed and this was repeated every 28 days during the period that the birds received the treatment (Table 1). During the experiment, information was collected on the daily production of eggs. Sample of the birds were weighed on a weekly basis.

### Feeding

The diet given to the birds was a standard feed for broiler chickens with the addition of the products that were tested. The feed met all nutritional requirements in relation to the developmental stage of the birds and the recommendations in the breeders' guide. The feed was entirely vegetable-based, using corn and Soya bran (Appendices 1, 2 and 3).

**TABLE 1. Treatments used in the experiment on broiler chickens (lineage - Cobb 500) for a period of six months.**

| Treatments | Carophyll Red (ppm) |
|---|---|
| 1 | 0 |
| 2 | 60 |

### Experimental Design

The experimental design was entirely random, with two treatments and six groups of 30 female and 3 male chickens.

### Methodology

The laying rate was calculated weekly. To assess the weight of the eggs, specific gravity, average weight of eggs, yolk weight, albumen weight and the coloration of the yolk, all the eggs that were not considered suitable for incubation, collected on any given day, were used. Specific gravity was determined through the emersion of the eggs in saline solutions with densities of 1065; 1070; 1075; 1080; 1085; 1090 and 1095. The weighing of the eggs, yolks and albumen were carried out using a precision weighing scale (0.001 g). The coloration of the yolks was determined using the color fan from DSM Nutritional Products^{®}.

To evaluate hatching, hatchability, fertility and embryonic mortality the eggs were collected daily. They were then classified and marked with the number of the corresponding box. Those that were not considered suitable for incubation were stored for a maximum period of seven days in an air-conditioned room with temperature and humidity control. Incubation was carried out in a multi-stage incubator and on day 18, the eggs were transferred to a brooder. On day 21 the chicks were taken out of the brooder, vaccinated and classified. The eggs that did not hatch then underwent embryo diagnostics to evaluate fertility and the phase of embryonic mortality.

### Statistical Analysis

After the data was obtained, a variation analysis was carried and standard deviation was calculated. These statistical procedures were carried out with the help of the statistical program SAS.

### Results

**TABLE 2. Laying rate during the periods 46th - 55th, 56th- 66th weeks and total period (21 weeks)**

| Treatments | Laying rate (%) | | |
|---|---|---|---|
| | 46th to 55th weeks | 56th to 66th week | Total period |
| Control | 58.19 ± 3.87 | 48.75 ± 3.19 | 53.25 ± 2.68 |
| Carophyll Red | 59.66 ± 2.97 | 51.35 ± 3.66 | 55.31 ± 2.22 |
| Arithmetic Mean | 58.93 | 50.05 | 54.28 |
| C.V. (%) | 5.86 | 6.87 | 4.54 |
| P | 0.4781 | 0.2207 | 0.1793 |

**TABLE 3. Hatchability in the periods between the 46th-56th and 56th-66th weeks and the total period (21 weeks)**

| Treatments | Hatchability (%) | | |
|---|---|---|---|
| | 46th to 55th week | 56th to 66th week | average of the 21 weeks |
| Control | 92.48 ± 0.98 b | 93.42 ± 0.51 b | 92.97 ± 0.54 b |
| Carophyll Red | 94.33 ± 0.76 a | 95.96 ± 0.53 a | 95.18 ± 0.56 a |
| Arithmetic Mean | 93.41 | 94.69 | 94.08 |
| C.V. (%) | 0.94 | 0.55 | 0.59 |
| P | 0.0047 | 0.0001 | 0.0001 |

| | | | |
|---|---|---|---|
| (a>b Duncan's test) | | | |

**TABLE 4. Hatching, hatchability, fertility, and embryonic mortality during the observed period (21 weeks)**

| Treatments | Hatching | Hatchability | Fertility | Embryonic Mortality |
|---|---|---|---|---|
| | (%) | | | |
| Control | 83.03 ± 0.89 b | 92.97 ± 0.54 b | 90.98 ± 0.81 b | 5.46 ± 0.75 a |
| Carophyll Red | 86.03 ± 0.42 a | 95.18 ± 0.56 a | 92.11 ± 0.48 a | 3.72 ± 0.86 b |
| Arithmetic Mean | 84.53 | 94.08 | 91.54 | 4.59 |
| C.V. (%) | 0.83 | 0.59 | 0.75 | 16.77 |
| P | 0.0001 | 0.0001 | 0.0171 | 0.0029 |

| | | | | |
|---|---|---|---|---|
| (a>b Duncan's test) | | | | |

**TABLE 5. Effect of the treatments on the average embryonic mortality rate over the observed period**

| Treatments | Embryonic Mortality (%) | | | |
|---|---|---|---|---|
| | M1 | M2 | M3 | M4 |
| Control | 1.80 ± 0.45 a | 0.89 ± 0.70 | 0.69 ± 0.28 | 2.07 ± 0.23 a |
| Carophyll Red | 1.04 ± 0.41 b | 0.66 ± 0.33 | 0.58 ± 0.33 | 1.44 ± 0.58 b |
| Arithmetic Mean | 1.42 | 0.77 | 0.64 | 1.76 |
| C.V. (%) | 28.02 | 40.41 | 54.07 | 21.86 |
| P | 0.0083 | 0.2225 | 0.5980 | 0.0171 |

| | | | | |
|---|---|---|---|---|
| (a>b Duncan's test) | | | | |

M 1- Embryonic Mortality in the first 48 hours of incubation
M 2- Embryonic Mortality occurring between day 3 and day 7 of incubation
M 3- Embryonic Mortality occurring between day 8 and day 14 of incubation
M 4- Embryonic Mortality occurring between day 15 and day 21 of incubation

**TABLE 6. Embryonic Mortality between the 19th and 21 st weeks and for all 21 weeks of the study**

| Treatments | Embryonic Mortality (%) | | | |
|---|---|---|---|---|
| | Week 19 | Week 20 | Week 21 | 21 weeks |
| Control | 5.32 ± 3.24 | 4.57 ± 1.57 | 5.18 ± 2.72 | 5.46 ± 0.75 a |
| Carophyll Red | 3.33 ± 4.12 | 3.51 ± 2.56 | 3.12 ± 2.12 | 3.72 ± 0.86 b |
| Arithmetic Mean | 4.33 | 4.04 | 4.15 | 4.59 |
| C.V. (%) | 57.93 | 52.75 | 54.88 | 16.77 |
| P | 0.0127 | 0.4081 | 0.1490 | 0.0029 |

| | | | | |
|---|---|---|---|---|
| (a>b Duncan's test) | | | | |

**TABLE 7. Number of eggs, incubatible eggs and chicks per bird housed during the observed period (21 weeks)**

| Treatments | Production of eggs/bird | Incubatible eggs/bird | Chicks/bird |
|---|---|---|---|
| Control | 78.27 ± 3.95 | 71.71 ± 4.46 | 5952 ± 3.18 b |
| Carophyll Red | 81.30 ± 3.40 | 75.25 ± 3.46 | 64.73 ± 2.82 a |
| Arithmetic Mean | 79.79 | 73.48 | 62.13 |
| C.V. (%) | 4.55 | 5.36 | 4.76 |
| P | 0.1793 | 0.1503 | 0.0122 |

**TABLE 8. Calculated nutritional levels in the feed used during the experiment**

| | | | | |
|---|---|---|---|---|
| Average Metabolic Energy (Kcal) | 2850.00 | | Average Metabolic Energy (Kcal) | 2850.00 |
| Gross Protein (%) | 15.96 | | Vitamin E (mg) | 53.59 |
| Arginine Total (%) | 0.94 | | Vitamin K3 (mg) | 2.50 |
| Lysine Total (%) | 0.80 | | Vitamin B1 (mg) | 5.77 |
| Methionine Total (%) | 0.35 | | Vitamin B2 (mg) | 10.82 |
| Methionine + Cystine Total (%) | 0.55 | | Vitamin B6 (mg) | 10.85 |
| Threonine Total (%) | 0.59 | | Vitamin B12 (mcg) | 19.00 |
| Triptophan Total (%) | 0.17 | | Biotin (mg) | 0.30 |
| Isoleucine Total (%) | 0.63 | | Folic Acid (mg) | 1.74 |
| Leucine Total (%) | 1.46 | | Nicotinic Acid (mg) | 65.36 |
| Valine Total (%) | 0.72 | | Pantothenic Acid (mg) | 25.22 |
| Histidine Total (%) | 0.42 | | Copper (mg) | 18.43 |
| Chlorine Total (%) | 0.77 | | Iron (mg) | 122.94 |
| Calcium (%) | 3.30 | | Iodine (mg) | 0.80 |
| Available Phosphorus (%) | 0.40 | | Manganese (mg) | 83.10 |
| Sodium (%) | 0.19 | | Selenium (mg) | 0.69 |
| Chlorine (%) | 0.28 | | Zinc (mg) | 93.83 |
| Potassium (%) | 0.60 | | | |
| Vitamin A (UI) | 10450.00 | | | |
| Canthaxanthin (UI) | 1662.50 | | | |

**TABLE 9. Composition of feed used during the experiment**

| Ingredients | Percentage |
|---|---|
| Corn | 68.52 |
| Soya Bran 46% | 21.57 |
| Limestone 38% Ca | 7.21 |
| Bicalcium Phosphate | 1.64 |
| Salt | 0.4 |
| Wheat Bran | 0.112 |
| DL-Methionine 99% | 0.045 |
| Premix | 0.50 |

**TABLE 10. Composition of the premix added to the diet of the chickens**

| Nutrient | Quantity per kilo of product | Unit |
|---|---|---|
| Folic Acid | 237.5 | mg |
| Nicotinic Acid | 8500 | mg |
| Pantothenic Acid | 3800 | mg |
| Biotin | 38 | mg |
| Copper | 12400 | mg |
| Choline | 72000 | mg |
| Sulphur | 10222 | mg |
| Iron | 12000 | mg |
| Iodine | 160 | mg |
| Manganese | 14000 | mg |
| Methionine | 118800 | mg |
| Oxytetracycline | 8000 | mg |
| Selenium | 108 | mg |
| Vitamin A | 2090000 | UI/Kg |
| Vitamin B1 | 475 | mg |
| Vitamin B12 | 3800 | mg |
| Vitamin B2 | 1900 | mg |
| Vitamin B6 | 950 | mg |
| Canthaxanthin | 332500 | UI/Kg |
| Vitamin E | 7600 | mg |
| Vitamin K3 | 950 | mg |
| Zinc | 14000 | mg |

### Example 2: Study if the Supplementation of Canthaxanthin and 25-OH D3 in broiler breeder hens

### Material & Methods:

The test has been executed according to Example 1, with the addition of ROVIMIX® Hy-D®. The treatments in example 2 were as follows:
T1 - Control diet
T2 - Control diet + 60 ppm of Carophyll Red
T3 - Control diet + 69 ppm of ROVIMIX® Hy-D®
T4 - Control diet + 60 ppm of Carophyll Red + 69 ppb of ROVIMIX® Hy-D® and with 6 replication/treatment of 40 broiler breeder and 4 cockerels per replication.

Egg production, fertility and hatchability are recorded weekly. The results are shown in Table 11. The data shows a good response for ROVIMIX® Hy-D® and canthaxanthin.

**TABLE 11:**

| TRT | Average hatchability after 10 weeks |
|---|---|
| Control | 85,26 |
| ROVIMIX® Hy-D® | 86,85 |
| CAROPHYLL RED | 87,22 |
| CAROPHYLL RED+ ROVIMIXO Hy-D® | 88,07 |

## Claims

1. The use of canthaxanthin for improving hatchability and fertility in poultry.

2. The use according to claim 1, wherein canthaxanthin is combined with at least one vitamin D metabolite.

3. The use according to claim 2, wherein the vitamin D metabolite is 25-hydroxy vitamin D3.

4. The use of canthaxanthin in the manufacture of a food or veterinary composition for lowering embryo mortality in poultry.

5. The use according to claim 4, wherein canthaxanthin is combined with at least one vitamin D metabolite.

6. The use according to claim 2, wherein the vitamin D metabolite is 25-hydroxy vitamin D3.

7. A poultry food comprising from about 10 mg/kg to about 100 mg/kg of 25-hydroxy vitamin D3 and from about 2 to 100 ppm canthaxanthin, preferably from about 2 ppm to 10 ppm.

8. A premix composition comprising canthaxanthin and 25-hydroxy vitamin D3, for use in breeder feed to lower embryo mortality.

9. Use of a premix composition comprising canthaxanthin and 25-hydroxy vitamin D3, for improving hatchability and fertility in poultry.

## Patentansprüche

1. Verwendung von Canthaxanthin zur Verbesserung der Schlupffähigkeit und Fruchtbarkeit bei Geflügel.

2. Verwendung nach Anspruch 1, wobei Canthaxanthin mit mindestens einem Vitamin-D-Metaboliten kombiniert wird.

3. Verwendung nach Anspruch 2, wobei es sich bei dem Vitamin-D-Metaboliten um 25-Hydroxy-Vitamin D3 handelt.

4. Verwendung von Canthaxanthin in der Herstellung eines Futters oder einer tierärztlichen Zusammensetzung für das Herabsetzen der Embryomortalität bei Geflügel.

5. Verwendung nach Anspruch 4, wobei Canthaxanthin mit mindestens einem Vitamin-D-Metaboliten kombiniert wird.

6. Verwendung nach Anspruch 4, wobei es sich bei dem Vitamin-D-Metaboliten um 25-Hydroxy-Vitamin D3 handelt.

7. Geflügelfutter, das ungefähr 10 mg/kg bis ungefähr 100 mg/kg 25-Hydroxy-Vitamin D3 und ungefähr 2 bis 100 ppm Canthaxanthin, vorzugsweise ungefähr 2 ppm bis 10 ppm, umfasst.

8. Premix-Zusammensetzung, die Canthaxanthin und 25-Hydroxy-Vitamin D3 umfasst, zur Verwendung in Brutgeflügelfutter zum Herabsetzen der Embryosterblichkeit.

9. Verwendung einer Premix-Zusammensetzung, die Canthaxanthin und 25-Hydroxy-Vitamin D3 zur Verbesserung der Schlupffähigkeit und Fruchtbarkeit bei Geflügel.

## Revendications

1. Utilisation de la canthaxanthine pour améliorer l'éclosabilité et la fécondité chez la volaille.

2. Utilisation selon la revendication 1, **caractérisée en ce que** la canthaxanthine est combinée avec au moins un métabolite de la vitamine D.

3. Utilisation selon la revendication 2, **caractérisée en ce que** le métabolite de la vitamine D est la 25-hydroxyvitamine D3.

4. Utilisation de la canthaxanthine dans la fabrication d'une composition alimentaire ou vétérinaire pour réduire la mortalité embryonnaire chez la volaille.

5. Utilisation selon la revendication 4, **caractérisée en ce que** la canthaxanthine est combinée avec au moins un métabolite de la vitamine D.

6. Utilisation selon la revendication 4, **caractérisée en ce que** le métabolite de la vitamine D est la 25-hydroxyvitamine D3.

7. Aliment pour volaille comprenant d'environ 10 mg/kg à environ 100 mg/kg de 25-hydroxyvitamine D3 et d'environ 2 à 100 ppm de canthaxanthine, de préférence d'environ 2 ppm à 10 ppm.

8. Composition de prémélange comprenant de la canthaxanthine et de la 25-hydroxyvitamine D3, destinée à être utilisée dans l'alimentation pour poulets d'élevage pour réduire la mortalité embryonnaire.

9. Utilisation d'une composition de prémélange comprenant de la canthaxanthine et de la 25-hydroxyvitamine D3 pour améliorer l'éclosabilité et la fécondité chez la volaille.
